(19) Europäisches Patentamt
European Patent Office
Office européen des brevets

(11) Veröffentlichungsnummer: **0 185 234**
**B1**

# (12) EUROPÄISCHE PATENTSCHRIFT

(45) Veröffentlichungstag der Patentschrift: **01.06.88**

(21) Anmeldenummer: **85115163.9**

(22) Anmeldetag: **29.11.85**

(51) Int. Cl.⁴: **C 07 C 69/732**, C 07 C 93/193, C 07 C 121/413, C 07 C 67/343, C 07 C 120/00

## (54) 2-(1-Hydroxycarbalkoxymethyl)-acrylnitril und -acrylester und Verfahren zu deren Herstellung.

(30) Priorität: **04.12.84 DE 3444097**

(43) Veröffentlichungstag der Anmeldung: **25.06.86 Patentblatt 86/26**

(45) Bekanntmachung des Hinweises auf die Patenterteilung: **01.06.88 Patentblatt 88/22**

(84) Benannte Vertragsstaaten: **CH DE FR GB IT LI**

(56) Entgegenhaltungen:
**FR - A - 2 120 686**

**TETRAHEDRON LETTERS, Band 25, Nr. 12, 1984, Seiten 1303-1306, Pergamon Press Ltd., GB; C. PAPAGEORGIOU et al.: "Synthesis of potentially allergenic chiral alpha-(hydroxyalkyl)acrylates" JOURNAL OF THE CHEMICAL SOCIETY, PERKIN TRANSACTIONS I, ORGANIC AND BIO-ORGANIC CHEMISTRY, 1973, Seiten 2016-2019; C.F: GARBERS et al.: "Stereospecific formation of a vinyl sulphide via a sulphonium ylide-salt coupling reaction"**

(73) Patentinhaber: **BASF Aktiengesellschaft, Carl-Bosch-Strasse 38, D-6700 Ludwigshafen (DE)**

(72) Erfinder: **Fikentscher, Rolf, Dr., Von-Stephan-Strasse 27, D-6700 Ludwigshafen (DE)**
Erfinder: **Hahn, Erwin, Dr., Am Buechsenackerhang 31, D-6900 Heidelberg (DE)**
Erfinder: **Kud, Alexander, Dr., Am Hellbrunn, D-6509 Eppelsheim (DE)**
Erfinder: **Oftring, Alfred, Dr., Berner Weg 26, D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Aus der US-PS 3 743 669 ist die Herstellung von 2-(1-Hydroxyalkyl)-acrylnitrilen und von 2-(1-Hydroxyalkyl)-acrylestern der allgemeinen Formel

RCHOH
|
$CH_2=C$-Y (III)

bekannt, in der Y u.a. = -CN oder

-C-OR$^1$
||
O

und R und R$^1$ eine Alkyl- oder Arylgruppe bedeuten. Die Verbindungen der Formel III werden hergestellt, indem man Acrylnitril oder einen Acrylester der Formel $CH_2$-CH-Y mit einem Aldehyd der Formel RCHO, wobei die Substituenten Y und R die in Formel III angegebene Bedeutung haben, in Gegenwart von tertiären Aminen als Katalysator bei Temperaturen von 0 bis 200°C reagieren lässt.

Aufgabe der Erfindung ist es, Verbindungen zur Verfügung zu stellen, die ausser einer Doppelbindung weitere funktionelle Gruppen im Molekül enthalten. Eine weitere Aufgabe besteht darin, ein Herstellverfahren für die neuen Verbindungen aufzuzeigen.

Die Aufgabe wird erfindungsgemäss gelöst mit einem Verfahren zur Herstellung von Verbindungen der Formel

H
RO-CO-C<
| OH
$CH_2=C$-X (I),

in der R = $C_1$- bis $C_{18}$-Alkyl,

X = -CN, -C-OR$^1$,
||
O

R$^1$ = -$(CH_2)_n$-OH, -$(CH_2)_m$-N<$^{R^2}_{R^3}$,

-$(CH_2)_m$-$\overset{\oplus}{N}$(R$^2$)(R$_3$)(R$^4$) $Y^{\ominus}$, -$(C_2H_4O)_p$R

R$^2$, R$^3$ = $-CH_3$, $-C_2H_5$

R$^4$ = -H, $-CH_3$, $-C_2H_5$, $-CH_2$– 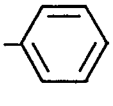

n = 2 bis 4,
m = 2 bis 5,
p = 1 bis 80 und
$Y^-$ = $Cl^-$, $Br^-$, $SO_4^{2-}$, $PO_4^{3-}$, $CH_3OSO_3^-$, $C_2H_5OSO_3^-$, $CH_3COO^-$, $HCOO^-$

bedeuten, wenn man eine Verbindung der Formel

H
|
$CH_2=C$-X (II),

in der X die in Formel I angegebene Bedeutung hat, mit Glyoxylsäureestern der Formel

RO-CO-CHO (III)

in der R = $C_1$- bis $C_{18}$-Alkyl bedeutet, in Gegenwart von tertiären Aminen als Katalysator umsetzt.

Als Verbindungen der Formel II kommen Acrylnitril und Acrylester in Betracht. Der Substituent X in Formel II kann folgende Bedeutung haben:

-CN, -C-OR$^1$,
||
O

R$^1$ = $C_1$- bis $C_{18}$-Alkyl, -$(CH_2)_n$-OH, -$(CH_2)_m$-N<$^{R^2}_{R^3}$,

-$(CH_2)_m$-$\overset{\oplus}{N}$(R$^2$)(R$_3$)(R$^4$) $Y^{\ominus}$, -$(C_2H_4O)_p$R

R = $C_1$- bis $C_{18}$-Alkyl
R$^2$, R$^3$ = $-CH_3$, $-C_2H_5$
R$^4$ = -H, $-CH_3$, $-C_2H_5$, $-CH_2$– 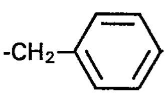

n = 2 bis 4,
m = 2 bis 5,
p = 1 bis 80 und
$Y^-$ = $Cl^-$, $Br^-$, $SO_4^{2-}$, $PO_4^{3-}$, $CH_3OSO_3^-$, $C_2H_5OSO_3^-$, $CH_3COO^-$, $HCOO^-$.

$C_1$ bis $C_{18}$ Alkylacrylate sind beispielsweise Methylacrylat, Ethylacrylat, n-Propylacrylat, Isopropylacrylat, n-Butylacrylat, Isobutylacrylat, tert.-Butylacrylat, n-Octylacrylat, iso-Octylacrylat, 2-Ethylhexylacrylat, Cyclopentylacrylat, Cyclohexylacrylat, Laurylacrylat, Palmitylacrylat und Stearylacrylat. Die Formel II umfasst ausserdem Hydroxyethylacrylat, Hydroxypropylacrylat und Hydroxybutylacrylat, wobei diejenigen Hydroxyalkylacrylate, die sich von $C_3$- und $C_4$-Glykolen ableiten, durch Veresterung von sämtlichen möglichen isomeren Glykolen oder deren Mischungen mit Acrylsäure hergestellt werden können, sowie Dimethylaminoethylacrylat, Diethylaminoethylacrylat, Dimethylaminopropylacrylat, Diethylaminopropylacrylat, Dimethylaminobutylacrylat, Diethylaminobutylacrylat, Dimethylaminoneopentylacrylat, Diethylaminoneopentylacrylat sowie die neutralisierten bzw. quaternierten Dialkylaminoalkylacrylate. Als Quaternierungsmittel kommen beispielsweise Dimethylsulfat, Diethylsulfat, Methylchlorid, Ethylchlorid, Methylbromid, Ethylbromid und Benzylchlorid in Betracht. Die Salze der Dimethylaminoalkylacrylate werden durch Neutralisieren mit Salzsäure, Bromwasserstoffsäure, Schwefelsäure, Phosphorsäure oder gesättigten Carbonsäuren, wie Ameisensäure und Essigsäure erhalten. Als Verbindungen der Formel II kommen auch Polyethoxyalkylester der Acrylsäure in Betracht, die 1 bis 80, vorzugsweise 3 bis 30 Ethoxygruppen enthalten, z.B. Methoxyethylacrylat, Ethoxyethylacrylat und Lauryloxitrioxyethylacrylat. Von den Verbindungen der Formel II werden vorzugsweise Acrylnitril, Methylacrylat, Ethylacrylat, 2-Ethylhexylacrylat, Hydroxyethylacrylat und Hydroxybutylacrylat eingesetzt.

Die Verbindungen der Formel II werden in dem Temperaturbereich von 0 bis 150, vorzugsweise von 0 bis 70 °C bei einem pH-Wert von 7 bis 13 in Gegenwart von tert. Aminen als Katalysator mit Glyoxylsäureestern der Formel III umgesetzt. Der pH-Wert des Reaktionsgemisches kann 7 bis 13 betragen und liegt vorzugsweise in dem Bereich von 8 bis 10. Er wird durch Zugabe der als Katalysator wirkenden tert. Amine und gegebenenfalls noch durch Zusatz von anderen Basen, wie Natronlauge, Kalilauge, Soda, Pottasche oder Calciumhydroxid eingestellt. In den Glyoxylsäureestern der Formel III kann R eine $C_1$- bis $C_{18}$-Alkylgruppen bedeuten. Geeignete Glyoxylsäureester sind beispielsweise Glyoxylsäuremethylester, Glyoxylsäureethylester, Glyoxylsäure-n-propylester, Glyoxylsäureisopropylester, Glyoxylsäurebutylester, Glyoxylsäure-2-ethylhexylester und Glyoxylsäurestearylester.

Die Umsetzung kann in Abwesenheit von Verdünnungsmitteln, d.h. in Reinsubstanz, oder in einem inerten Lösemittel durchgeführt werden. Als inerte Lösemittel eignen sich beispielsweise Ether, wie Diethylether, Dioxan, Tetrahydrofuran, Dimethylglykolether, Diethylglykolether, Methylglykol, Ethylglykol, Polyglykolether, ausserdem Acetonitril, aliphatische und aromatische Kohlenwasserstoffe (z.B. Pentan, Hexan, Cyclohexan, i-Octan, n-Octan, Dodecan, Toluol, Xylol und Tetralin), chlorierte Kohlenwasserstoffe (z.B. Dichlormethan, Tetrachlorkohlenstoff, Trichlorethan und Tetrachlorethan), Methylethylketon, Aceton und Wasser. In einigen Fällen kann die Verwendung eines Lösemittelgemisches gegenüber einem einzigen Lösemittel Vorteile bieten.

Die Reaktion wird vorzugsweise in homogener Mischung und in Abwesenheit von inerten Lösemitteln durchgeführt. Sofern man Acrylester und Glyoxylsäureester verwendet, die sich jeweils in Wasser nur sehr schlecht lösen, empfiehlt es sich, die Umsetzung in Gegenwart eines gegenüber den Reaktionsteilnehmern inerten Lösemittels durchzuführen, das sowohl mit den Reaktionsteilnehmern und mit Wasser mischbar ist. Auf 100 Gew.-Teile der Mischung aus den Verbindungen der Formeln II und III kann man bis zu 1.000, vorzugsweise 10 bis 200 Gew.-Teile eines inerten Lösemittels einsetzen.

Die Umsetzung erfolgt in Gegenwart von tertiären Aminen. Man kann sowohl offenkettige aliphatische Amine als auch cyclische tertiäre Amine verwenden. Beispiele hierfür sind: Trimethylamin, Triethylamin, Tri-n-propylamin, Triisopropylamin, Tributylamin, Triisobutylamin, Tri-n-pentylamin, N-Methyldiisopropylamin, N,N-Diethylisopropylamin, N,N-Dimethylethylamin, N,N-Dimethylisopropylamin, Tri-2-ethylhexylamin, N-Methyldiethylamin, N,N-Dimethyl-n-propylamin, N,N-Dimethyl-n-butylamin, N,N-Dimethyl-isobutylamin, N,N-Dimethyl-(2-ethylhexyl)amin, N,N-Diisopropyl-(2-ethylhexyl)-amin, N,N-Di-n-butyl-(2-ethylhexyl)-amin, N-Methyl-di-(2-ethylhexyl)amin, N-n-butyl-di-(2-ethylhexyl)-amin, N-Isobutyl-di(2-ethylhexyl)-amin, 1,1-Diazabicyclo-[2,2,2]-octan, Pyrrocolin und Chinolidin. Bezogen auf 100 Gewichtsteile der Mischung aus den Verbindungen der Formel II und Formaldehyd setzt man 0,1 bis 10, vorzugsweise 3 bis 8 Gewichtsteile eines tertiären Amins ein. Die Umsetzung wird bevorzugt in Gegenwart von 1,4-Diazabicyclo-[2,2,2]-octan, Pyrrocolin und Chinolidin durchgeführt. Die Verbindungen der Formel II und III werden in aller Regel in molarem Verhältnis miteinander zur Reaktion gebracht. Technische Gegebenheiten können es jedoch erforderlich machen, die eine oder die andere Komponente im Überschuss einzusetzen. So ist es beispielsweise möglich, das molare Verhältnis von Glyoxylsäureestern zu den Verbindungen der Formel II in dem Bereich von 20:1 bis 1:2, vorzugsweise 5:1 bis 0,75:1 zu variieren. Die Reaktion wird üblicherweise bei Atmosphärendruck durchgeführt. Sie kann jedoch auch bei vermindertem oder erhöhtem Druck vorgenommen werden. Ein Arbeiten unter Druck ist vor allem dann erforderlich, wenn die Umsetzung bei Temperaturen oberhalb von 100 °C vorgenommen wird.

Die nach dem erfindungsgemässen Verfahren hergestellten Reaktionsprodukte — 2-(1-Hydroxycarbalkoxymethyl)-acrylnitrile und -acrylester der oben angegebenen Formel I — sind wertvolle Zwischenprodukte, die sich beispielsweise als Monomer zur Herstellung von Polymerisaten eignen.

*Beispiel 1*

102 g (1 Mol) Glyoxylsäureethylester, 86 g (1 Mol) Acrylsäuremethylester und 7 g (0,062 Mol) 1,4-Diazabicyclo-[2,2,2]-octan (im folgenden Text mit DABCO abgekürzt) werden 48 Stunden in einem 2 l-Dreihalskolben in einer Stickstoffatmosphäre bei 50 °C kräftig gerührt. Danach entfernt man die flüchtigen Anteile im Wasserstrahlvakuum, nimmt den Rückstand mit 250 ml Diethylether auf, wäscht die resultierende Lösung mit 60 ml 8%iger wässriger Salzsäure und anschliessend mir 100 ml Wasser. Die organische Phase wird dann über Natriumsulfat getrocknet, der Ether entfernt und der Rückstand in Vakuum destilliert. Man erhält 149 g (79% d.Th.) 2-(1-Hydroxy-carbethoxymethyl)-methylacrylat als farblose Flüssigkeit mit einem Siedepunkt von 96 bis 100 °C bei 0,4 mbar.

*Beispiel 2*

26 g (0,2 Mol) Glyoxylsäure-n-butylester, 25,6 g (0,2 Mol) iso-Butylacrylat und 2 g (17,8 mMol) DABCO werden 27 h unter Rühren auf 50 °C erwärmt. Anschliessend werden flüchtige Anteile im Vakuum entfernt, der ölige Rückstand wird in 200 ml Diethylether aufgenommen, die Lösung mit 50 ml 10%iger wässriger Salzsäure und mit 50 ml $H_2O$ gewaschen. Nach dem Trocknen über Natriumsulfat wird das Lösemittel abdestilliert. Es verbleibt ein gelbes Öl: 34 g (66% d.Th.) 2-(1-Hydroxy-carb-butoxymethyl)-isobutyl-acrylat.

*Beispiel 3*

44 g (0,5 Mol) Glyoxylsäuremethylester, 43 g (0,5 Mol) Methylacrylat und 4 g (35,7 mMol) DABCO werden 48 h bei 25 °C kräftig gerührt. Nach dem Abdestillieren flüchtiger Anteile im Wasserstrahlvakuum wird der ölige Rückstand in 200 ml Diethylether aufgenommen und die resultierende Lösung

mit 50 ml 8%iger Salzsäure und mit 75 ml Wasser gewaschen. Die organische Phase wird nach Trocknen über Natriumsulfat und Entfernen des Lösungsmittels im Ölpumpenvakuum fraktionierend destilliert. Man erhält 64 g (74% d.Th.) 2-(1-Hydroxy-carbomethoxymethyl)-methylacrylat vom Siedepunkt 83-84°C (0,4 mbar).

*Beispiel 4*

10,2 (0,1 Mol) Glyoxylsäureethylester, 10 g (0,1 Mol) Ethylacrylat und 1 g (8,9 mMol) DABCO werden 48 h bei 60°C kräftig gerührt. Danach werden flüchtige Anteile im Wasserstrahlvakuum entfernt, der Rückstand wird in 50 ml Diethylether aufgenommen und die resultierende Lösung mit 20 ml 8%iger Salzsäure und anschliessend mit 30 ml Wasser gewaschen. Nach dem Trocknen der organischen Phase über Natriumsulfat und dem Abtrennen des Lösungsmittels erhält man durch fraktionierende Destillation des Rückstandes 11 g (54% d.Th.) 2-(1-Hydroxy-carbethoxymethyl)-ethylacrylat vom Siedepunkt 120-122°C (0,3 mbar).

Analoge Versuchsführung mit Triethylamin anstelle von DABCO führt zu einer Ausbeute von 34% d.Th.

*Beispiel 5*

20,4 g (0,2 Mol) Glyoxylsäureethylester, 10,6 g (0,2 Mol) Acrylnitril und 2 g (17,8 mMol) DABCO werden 70 Stunden bei 25°C intensiv gerührt. Die Aufarbeitung erfolgt analog zu der unter Beispiel 2 beschriebenen. Der Rückstand wird einer fraktionierten Destillation unterworfen. Bei einem Druck von 0,5 mbar erhält man im Temperaturbereich von 105-107°C 17 g (51% d.Th.) 2-(1-Hydroxycarboethoxymethyl)-acrylnitril in Form einer farblosen Flüssigkeit.

*Beispiel 6*

51 g (0,5 Mol) Glyoxylsäureetkylester, 58 g (0,5 Mol) Hydroxyethylacrylat und 5 g (44,6 mMol) DABCO werden mit 100 ml absolutem Acetonitril vermischt. Die resultierende Lösung wird 70 Stunden bei 25°C gerührt. Die Aufarbeitung erfolgt analog zu der unter Beispiel 2 beschriebenen. Man erhält ein schwach gelbes Öl: 63 g (58% d.Th.) 2-(1-Hydroxy-carbethoxymethyl)-hydroxyethylacrylat.

*Beispiel 7*

Es werden 51 g (0,5 mol) Glyoxylsäureethylester, 85,5 g (0,5 Mol) N,N-Diethylaminoethylacrylat und 7 g (62,5 mMol) DABCO in 100 ml absolutem Dioxan analog zu Beispiel 6 umgesetzt. Man erhält 74 g (54% d.Th.) 2-(1-Hydroxycarbethoxymethyl)-diethylaminoethylacrylat.

**Patentansprüche**

1. Verfahren zur Herstellung von Verbindung der Formel

$$RO\text{-}CO\text{-}C(H)(OH)\text{-}C(=CH_2)\text{-}X \quad (I),$$

in der R = $C_1$- bis $C_{18}$-Alkyl,

$$X = \text{-CN},\ \text{-C(=O)-OR}^1,$$

$$R^1 = \text{-(CH}_2)_n\text{-OH},\ \text{-(CH}_2)_m\text{-N}(R^2)(R^3),$$

$$\text{-(CH}_2)_m\text{-}\overset{\oplus}{N}(R^2)(R_3)(R^4)\ Y^{\ominus},\quad \text{-(C}_2H_4O)_pR$$

$R^2$, $R^3$ = $-CH_3$, $-C_2H_5$

$R^4$ = -H, $-CH_3$, $-C_2H_5$, $-CH_2-$ 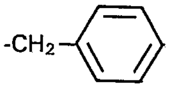

n = 2 bis 4,
m = 2 bis 5,
p = 1 bis 80 und
$Y^-$ = $Cl^-$, $Br^-$, $SO_4^{2-}$, $PO_4^{3-}$, $CH_3OSO_3^-$, $C_2H_5OSO_3^-$, $CH_3COO^-$, $HCOO^-$

dadurch gekennzeichnet, das man eine Verbindung der Formel

$$CH_2{=}C(H)\text{-}X \quad (II),$$

in der X die in Formel I angegebene Bedeutung hat, mit Glyoxylsäureestern der Formel

$$RO\text{-}CO\text{-}CHO \quad (III),$$

in der R = $C_1$- bis $C_{18}$-Alkyl bedeutet, in Gegenwart von tertiären Aminen als Katalysator umsetzt.

2. Verfahren nach Anspruch 1, dadurch gekennzeichnet, dass man die Umsetzung bei einer Temperatur von 0 bis 150°C durchführt.

3. Verfahren nach den Ansprüchen 1 und 2, dadurch gekennzeichnet, dass man die Umsetzung bei einem pH-Wert von 7 bis 13, durchführt.

4. Verfahren nach den Ansprüchen 1 bis 3, dadurch gekennzeichnet, dass man die Umsetzung in Gegenwart eines inerten Lösemittels durchführt, in dem die Verbindungen II und III sowie Wasser löslich sind.

5. Verfahren nach den Ansprüchen 1 bis 4, dadurch gekennzeichnet, dass man, bezogen auf 100 Gew.-Teile der Mischung aus den Verbindungen der Formeln II und III, 10 bis 200 Gew.-Teile Dioxan, Tetrahydrofuran, Wasser und/oder Acetonitril als Lösemittel einsetzt.

6. Verfahren nach den Ansprüchen 1 bis 5, dadurch gekennzeichnet, dass man, bezogen auf 100 Gew.-Teile der Mischung aus den Verbindungen der Formeln II und III, 0,1 bis 10 Gew.-Teile eines tertiären Amins einsetzt.

7. Verfahren nach den Ansprüchen 1 bis 6, dadurch gekennzeichnet, dass man als tertiäre Amine 1,4-Diazabicyclo-[2,2,2]-octan, Pyrrocolin oder Chinolidin einsetzt.

8. Verfahren nach den Ansprüchen 1 bis 6, dadurch gekennzeichnet, dass man die Verbindungen III und II im molaren Verhältnis von 20:1 bis 1:2 umsetzt.

9. Verfahren nach den Ansprüchen 1 bis 4, dadurch gekennzeichnet, dass man die Verbindungen III und II im molaren Verhältnis von 1:1 umsetzt.

## Claims

1. A process for the preparation of a compound of the formula

$$RO\text{-}CO\text{-}C(H)(OH)\text{-}C(=CH_2)\text{-}X \quad (I)$$

where R is $C_1$- $C_{18}$-alkyl,

X is -CN, or $-C(=O)-OR^1$,

$R^1$ is $C_1$-$C_{18}$-alkyl, $-(CH_2)_n-OH$, $-(CH_2)_m-N(R^2)(R^3)$

$-(CH_2)_m-N^{\oplus}(R^2)(R_3)(R^4)$ $Y^{\ominus}$ or $-(C_2H_4O)_pR$

$R^2$, und $R^3$ are each $-CH_3$ or $-C_2H_5$,

$R^4$ is -H, $-CH_3$, $-C_2H_5$ or $-CH_2-$ 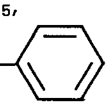

n is from 2 to 4,

m is from 2 to 5,

p is from 1 to 80, and

$Y^-$ is $Cl^-$, $Br^-$, $SO_4^{2-}$, $PO_4^{3-}$, $CH_3OSO_3^-$, $C_2H_5OSO_3^-$, $CH_3COO^-$, or $HCOO^-$

wherein a compound of the formula

$$CH_2=C(H)\text{-}X \quad (II),$$

where X has the meanings stated for formula I, is reacted with a glyoxylate of the formula

$$RO\text{-}CO\text{-}CHO \quad (III)$$

where R is $C_1$-$C_{18}$-alkyl, in the presence of a tertiary amine as catalyst.

2. A process as claimed in claim 1, wherein the reaction is carried out at from 0 to 150°C.

3. A process as claimed in claim 1 and 2, wherein the reaction is carried out at pH 7-13.

4. A process as claimed in Claim 1 to 3, wherein the reaction is carried out in the presence of an inert solvent in which the compounds II and III and water are soluble.

5. A process as claimed in claim 1 to 4, wherein from 10 to 200 parts by weight of dioxane, tetrahydrofuran, water and/or acetonitrile are used as solvent per 100 parts by weight of the mixture of the compounds of the formulae II and III.

6. A process as claimed in claim 1 to 5, wherein from 0.1 to 10 parts by weight of a tertiary amine are used per 100 parts by weight of the mixture of the compounds of the fromulae II and III.

7. A process as claimed in claim 1 to 6, wherein the tertiary amine used is 1,4-diazabicyclo[2,2,2]-octane, pyrrocoline or quinolidine.

8. A process as claimed in claim 1 to 6, wherein the compounds III and II are reacted in a molar ratio of from 20:1 to 1:2.

9. A process as claimed in claims 1 to 6,, wherein the compounds III and II are reacted in a molar ratio of 1:1.

## Revendications

1. Procédé de préparation de composés de formule

$$RO\text{-}CO\text{-}C(H)(OH)\text{-}C(=CH_2)\text{-}X \quad (I)$$

dans laquelle R = alkyle en $C_1$- à $C_{18}$

X = -CN, $-C(=O)-OR^1$,

$R^1$ = $-(CH_2)_n-OH$, $-(CH_2)_m-N(R^2)(R^3)$

$-(CH_2)_m-N^{\oplus}(R^2)(R_3)(R^4)$ $Y^{\ominus}$, $-(C_2H_4O)_pR$

$R^2$, $R^3$ = $-CH_3$, $-C_2H_5$

$R^4$ = -H, $-CH_3$, $-C_2H_5$, $-CH_2-$ 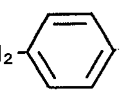

n = 2 à 4,

m = 2 à 5,

p = 1 à 80 et

$Y^-$ = $Cl^-$, $Br^-$, $SO_4^{2-}$, $PO_4^{3-}$, $CH_3OSO_3^-$, $C_2H_5OSO_3^-$, $CH_3COO^-$, $HCOO^-$

caractérisé par le fait que l'on fait réagir un composé de formule

$$CH_2=C(H)\text{-}X \quad (II),$$

dans laquelle X a la signification donnée dans la formule I, avec un ester d'acide glyoxylique de formule

$$RO\text{-}CO\text{-}CHO \quad (III),$$

dans laquelle R représente alkyle en $C_1$ à $C_{18}$ , en présence d'amines tertiaires comme catalyseur.

2. Procédé selon la revendication 1, caractérisé par le fait que l'on effectue la réaction à une température de 0 à 150°C.

3. Procédé selon la revendication 1 et 2, caractérisé par le fait que l'on effectue la réaction à un pH de 7 à 13.

4. Procédé selon la revendication 1 à 3, caracté-

risé par le fait que l'on effectue la réaction en présence d'un solvant inerte dans lequel sont solubles les composés II et III ainsi que l'eau.

5. Procédé selon la revendication 1 à 4, caractérisé par le fait que l'on introduit, comme solvant, 10 à 200 parties en poids de dioxane, tétrahydrofurane, eau et/ou acétonitrile, par rapport à 100 parties en poids du mélange des composés de formule II et III.

6. Procédé selon les revendications 1 à 5, caractérisé par le fait que l'on introduit 0,1 à 10 parties en poids d'une amine tertiaire, par rapport à 100 parties en poids du mélange des composés de formule II et III.

7. Procédé selon la revendication 1 à 6, caractérisé par le fait que l'on introduit, comme amine tertiaire, 1,4-diazabicyclo-[2,2,2]-octane, pyrrocoline ou quinolidine.

8. Procédé selon les revendications 1 à 6, caractérisé par le fait que l'on fait réagir les composés III et II dans un rapport molaire de 20/1 à 1/2.

9. Procédé selon les revendications 1 à 6, caractérisé par le fait que l'on fait réagir les composés III et II dans un rapport molaire de 1/1.